# EUROPEAN PATENT APPLICATION

(11) **EP 2 875 777 A1**
(43) Date of publication of application: **27.05.2015**
(21) Application number: 14003998.3
(22) Date of filing: 26.11.2014
(51) Int. Cl.: A61B 5/00, G01F 1/36, F15D 1/00

(54) **Flow sensing device**

(30) Priority: 26.11.2013 GB 201320883
(71) Applicant: OES Medical Limited, Witney, Oxfordshire OX29 7QG (GB)
(72) Inventor: Fiedorowicz, Richard, Abingdon, Oxfordshire OX13 6ER (GB)
(74) Representative: Franks & Co (South) Limited

(57) **Abstract**

A fluid sensor (1) consists of a hollow cylindrical casing (2) containing a large number of solid spheres (3) of identical diameter, packed tightly together. Fluid inflow and outflow blocks (4, 5) are mounted to each end of the casing (2), forming a fluid-tight seal. The inflow and outflow blocks (4, 5) each enclose a generally conical fluid chamber (6, 8) tapering from where it meets an end of an interior of the casing (2) to a respective inlet or outlet passage (7, 9). Circular grilles (10) divide the casing (2) from each fluid chamber (6, 8) and retain the spheres (3) in place. When a fluid, such as an anaesthetic gas mixture, is passed through the sensor (1) from the inlet to the outlet passage (7, 9), it must follow an indirect path between the spheres (3) packed into the casing (2). There is hence a resistance to the flow, which is manifested as a pressure differential across the casing (2). The pressure differential is measured via side passages (13) extending laterally from each fluid chamber (6, 8). For a given fluid and a given casing and sphere diameter, this pressure differential can be converted to a fluid flow rate.

## Description

The present invention relates to the area of devices for determining fluid flow rates. More particularly, but not exclusively, it relates to a device for determining flow rates of gases in anaesthetic ventilators.

Many forms of equipment require a flow rate of liquids, gases or vapours through the equipment to be measured, whether purely for recording purposes or so that deviations from a desired fluid flow rate may be identified and corrected. A wide range of flow rate measuring devices are known, but many are suitable only for a limited range of fluid viscosities and cannot easily be adjusted for use with fluids having viscosities outside this range.

For example, in the field of anaesthetic ventilators, a general idea of flow rates can be obtained by passing a carrier gas or vapour flow through a restricted orifice and determining a pressure drop across the orifice. However, this approach has been found to be insufficiently reliable. For example, the exact configuration and size of the orifice can be critical, leading to poor reproductibility between different units of apparently identical design. A particular orifice size is only effective for a particular, usually narrow, range of fluid viscosities. Additionally, flow through such an orifice can become non-linear or even turbulent at high fluid flow rates, and a restricted orifice may also significantly restrict the fluid flow rates that it is intended to measure.

Similar issues are believed to apply to other fluid flow measuring devices in a range of different fields, working with a range of different fluids.

It is hence an object of the present invention to provide a fluid flow sensing and measuring device that has greater flexibility and adaptability than existing devices, and which obviates some or all of the above drawbacks of existing devices.

According to a first aspect of the present invention, there is provided a fluid flow sensing device comprising chamber means extending from a first opening means at a first end of the chamber means to a second opening means at a second end of the chamber means remote from the first, a plurality of substantially identical bodies so packed into said chamber means as to define a plurality of indirect fluid passages extending between said first and second opening means, means to direct a flow of fluid to be measured through the chamber means and means to determine a differential pressure between fluid adjacent the first end of the chamber means and fluid adjacent the second and thereof.

Preferably, the flow sensing device comprises means to calculate a fluid flow rate from said differential pressure.

Alternatively, the fluid flow rate may be determined by an operator of the device, optionally using predetermined graphs, tables or other conversion means between differential pressure and fluid flow rate.

Preferably, the chamber means has a substantially constant cross-section between said first and second ends.

In a preferred embodiment, the chamber means comprises an elongate hollow cylindrical vessel, said first and second opening means being located at longitudinally remote ends of the vessel.

Said first and second opening means may then each comprise a respective open end of said vessel.

Preferably, each of said plurality of substantially identical bodies comprises a solid body.

Advantageously, each of said plurality of substantially identical bodies is substantially spherical.

Each said substantially spherical body may have the same preselected body diameter.

Preferably, said plurality of substantially identical bodies substantially fills an interior of the chamber means.

Said substantially identical bodies may be substantially regularly packed within the chamber means.

Alternatively, said substantially identical bodies may be substantially randomly packed within the chamber means.

The substantially identical bodies may be so packed as to approximate a close packing arrangement.

Preferably, the means to direct a flow of fluid through the chamber means comprises fluid inlet means operatively connected to the first opening means and fluid outlet means operatively connected to the second opening means.

Each of said fluid inlet and outlet means may comprise plenum chamber means, contiguous at one end with a respective opening means of the chamber means.

Preferably, said means to determine a differential pressure is operatively connected between said fluid inlet means and said fluid outlet means.

Advantageously, said means to determine a differential pressure is operatively connected between said plenum chamber means through aperture means extending through a respective wall of each said plenum chamber means.

Preferably, said first and second opening means of the chamber means are each provided with means to retain the plurality of substantially identical bodies within the chamber means.

Each said retaining means may comprise grille means having an aperture size below a minimum dimension of each said body.

Said grille means may comprise at least one aperture means having a profile differing substantially from a cross-sectional profile of each said body, so as to avoid said aperture means being blocked by a body in contact therewith.

Preferably, an average diameter of each said substantially identical body is between one third and one twentieth of a diameter of the chamber means orthogonal to an axis extending between its respective first and second opening means.

Advantageously, said average diameter of the bodies is less than one quarter of said chamber diameter.

Said average diameter of the bodies may be at least one tenth of said chamber diameter.

Optionally, said average diameter of the bodies may be less than one fifth of said chamber diameter.

Optionally, said average diameter of the bodies may be at least one eighth of said chamber diameter.

In a preferred embodiment, the fluid flow sensing device is so configured that said plurality of substantially identical bodies may be removed and replaced with a second plurality of substantially identical bodies, for example having a different diameter.

The chamber means may then be provided with selectably removable retaining means.

The fluid inlet and/or outlet means may then be selectably detachable from the chamber means, so as to allow access to a respective opening means.

Preferably, the fluid flow sensing device comprises a gas flow sensing device; i.e. said fluid comprises a gas.

According to a second aspect of the present invention, there is provided anaesthetic ventilator apparatus, provided with fluid flow sensing means as described in the first aspect above.

An example of the present invention will now be more particularly described, by way of example and with reference to the Figures of the accompanying drawings, in which
**Figure 1** is an exploded isometric view of a fluid flow sensor embodying the present invention; and
**Figure 2** is a longitudinal cross-sectional view of the fluid flow sensor of Figure 1.

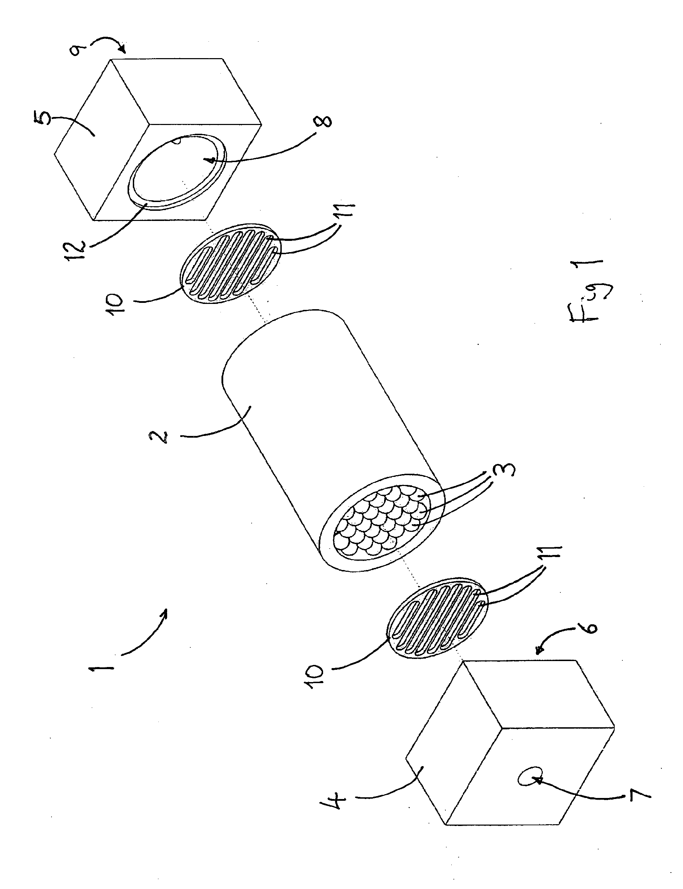

Referring now to the Figures, and to Figure 1 in particular, a fluid flow sensor 1 embodying the present invention comprises a hollow metal cylindrical casing 2 filled with a plurality of solid metal balls 3, each of identical diameter. Ideally, the balls 3 are of stainless steel.

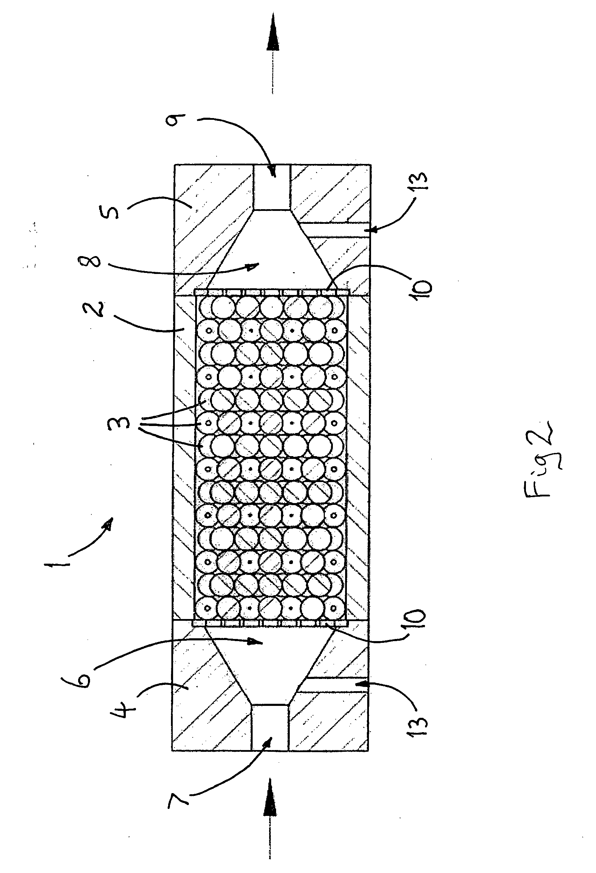

In this embodiment, the balls 3 are shown packed in a regular, layered array (see also Figure 2). However, it had been found that a more important consideration is that the balls 3 should be packed tightly and that the cylindrical casing 2 is filled with as many balls as possible. A perfectly regular geometrical array is not essential, and random or pseudorandom close packing appears to be sufficient.

The cylindrical casing 2 may thus conveniently be filled as follows. Balls 3 are poured into the casing 2 until it appears full, and the casing 2 is then tapped to settle the packing of the balls 3. The resulting free volume released within the casing 2 is then topped-up with a few further balls 3, filling the casing 2 to a maximum practical extent.

In the illustrated embodiment, the diameter of each ball 3 is between one-sixth and one-seventh of an internal diameter of the cylindrical casing 2. Larger or smaller balls 3 may be used, with effects on the performance of the fluid flow sensor 1 as described below. However, as the diameter of the balls 3 becomes a larger proportion of the internal diameter of the cylindrical casing 2, producing a tight packing becomes increasingly difficult.

A fluid inflow block 4 is mounted to the first end of the cylindrical casing 2, and a fluid outflow block 5 is mounted to a second end of the cylindrical casing 2, remote from the first. Each of the fluid inflow block 4 and the fluid outflow block 5 makes a fluid-tight seal with the respective end of the cylindrical casing 2.

The fluid inflow block 4 is provided with a generally conical fluid chamber 6 (not visible in this view), diverging from a coaxial inlet passage 7 towards an open mouth of the chamber 6. Said open mouth corresponds substantially in diameter to the internal diameter of the cylindrical casing 2, with which it is coaxially aligned.

Similarly, the fluid outflow block 5 is provided with a generally conical fluid chamber 8 that converges from its open mouth towards a coaxial outflow passage 9 (not visible in this view). Said open mouth also corresponds substantially in diameter to the internal diameter of the cylindrical casing 2, and is aligned coaxially therewith.

The fluid inflow block 4 and the fluid outflow block 5 are each fitted with a circular grille 10, having a plurality of elongate, narrow, parallel slots 11 extending through it, and seated in an annular recess 12 extending around a rim of the open mouth of each respective fluid chamber 6, 8.

When the respective fluid inflow and outflow blocks 4, 5 are fitted to respective ends of the cylindrical casing 2, these grilles 10 thus prevent the balls 3 escaping from the cylindrical casing 2. The elongate slots 12 have this shape to avoid balls 3 blocking the slots 12, and so blinding the grille 10.

Referring now to Figure 2, the packing of the balls 3 within the cylindrical casing 2 can more clearly be seen, as can the respective fluid chambers 6, 8 and inlet/outlet passages 7,9 of the fluid inflow and outflow blocks 4,5.

Fluid, such as air, a carrier gas, a vapour-laden carrier gas or even a liquid, may thus be passed onto the fluid flow sensor 1, through the inlet passage 7 and the fluid chamber 6, along the cylindrical casing 2 between the balls 3, and out through the fluid chamber 8 and the outlet passage 9.

The fluid inflow block 4 and the fluid outflow block 5 each have a side passage 13 extending radially outwardly from the respective conical fluid chambers 6,8. These side passages 13 are each connected to a pressure sensor arrangement, which detects a differential pressure between the fluids in the respective fluid chambers 6, 8. This differential pressure is sufficiently close, for the purposes of the present invention, to a differential pressure across the cylindrical casing 2.

There are no direct paths for fluid through the cylindrical casing 2 from end to end. Because of the balls 3 packed into the cylindrical casing 2, there are, however, a large number of indirect paths that fluid may follow, the cross-sectional dimensions of these indirect paths being closely linked to the diameter of the balls 3. There will thus be a resistance to flow of the fluid, which depends on the dimensions of the indirect paths (and hence those of the balls 3), on the viscosity of the fluid, and on the overall fluid flow rate. This resistance causes the differential pressure that can be measured across the cylindrical casing 2.

Since the ball 3 size will be constant, as will the fluid viscosity (as long as the local temperature remains reasonably constant), the differential pressure can be related directly to the flow rate of fluid through the fluid flow sensor 1.

It has been found that the fluid flow sensor 1 described can accommodate a wide range of flow rates without adjustment, since the flow of the fluid between the balls 3 remain laminar. A particular fluid flow sensor 1 can easily be modified to measure flow rates in a different range, and/or to measure flows of a fluid having a different viscosity, by removing a fluid inflow or outflow block 4, 5 and associated grille 10, removing the particular balls 3 in use, and replacing them with a packing of balls 3 having a different diameter.

After reassembly, the fluid flow sensor 1 may be used as described above, while substituting appropriate parameters such as ball diameter and fluid viscosity into the conversion of differential pressure to fluid flow rate.

It is also envisaged that alternative embodiments with different cylindrical casing 2 diameters and/or lengths could be used. These will also operate over different fluid flow rate ranges, and can also be used with balls 3 having different absolute sizes and/or different sizes relative to the cylindrical casing 2.

The repeatability of results between different units has been found to be excellent.

The benefits of the fluid flow sensor 1 described have so far been found mainly in monitoring the flow of gases and vapours, for example in anaesthetic ventilators and the like. However, there are indications that the same approach is equally suitable for liquids, only needing to allow for their higher viscosities.

The number of alternative paths through the cylindrical casing 2, around the balls 3, means that blockages are far less likely to occur than for existing systems using a single orifice. Also, the fluid flow sensor 1 of the present invention is far less likely to restrict the fluid flow that is being monitored, compared with the single narrow orifice of existing systems.

## Claims

1. A fluid flow sensing device (1), **characterised in that** the device comprises: chamber means (2) extending from a first opening means at a first end of the chamber means (2) to a second opening means at a second end of the chamber means (2) remote from the first; a plurality of substantially identical bodies (3) so packed into said chamber means (2) as to define a plurality of indirect fluid passages extending between said first and second opening means; means (4, 5) to direct a flow of fluid to be measured through the chamber means (2); and means to determine a differential pressure between fluid adjacent the first end of the chamber means (2) and fluid adjacent the second end (2) thereof.

2. A fluid flow sensing device (1) as claimed in claim 1, **characterised in that** the flow sensing device (1) comprises means to calculate a fluid flow rate from said differential pressure.

3. A fluid flow sensing device (1) as claimed in either claim 1 or claim 2, **characterised in that** the chamber means (2) has a substantially constant cross-section between said first and second ends.

4. A fluid flow sensing device (1) as claimed in any one of the preceding claims, **characterised in that** the chamber means (2) comprises an elongate hollow cylindrical vessel (2), said first and second opening means being located at longitudinally remote ends of the vessel (2).

5. A fluid flow sensing device (1) as claimed in any one of the preceding claims, **characterised in that** each of said plurality of substantially identical bodies (3) comprises a solid body (3)

6. A fluid flow sensing device (1) as claimed in any one of the preceding claims, **characterised in that** each of said plurality of substantially identical bodies (3) is substantially spherical.

7. A fluid flow sensing device (1) as claimed in any one of the preceding claims, **characterised in that** each said substantially spherical body (3) has the same preselected body diameter.

8. A fluid flow sensing device (1) as claimed in any one of the preceding claims, **characterised in that** said plurality of substantially identical bodies (3) substantially fills an interior of the chamber means (2).

9. A fluid flow sensing device (1) as claimed in any one of the preceding claims, **characterised in that** the means to direct a flow of fluid through the chamber means (2) comprises fluid inlet means (4) operatively connected to the first opening means and fluid outlet means (5) operatively connected to the second opening means.

10. A fluid flow sensing device (1) as claimed in claim 9, **characterised in that** said means to determine a differential pressure is operatively connected between said fluid inlet means (4) and said fluid outlet means (5).

11. A fluid flow sensing device (1) as claimed in any one of the preceding claims, **characterised in that** said first and second opening means of the chamber means (2) are each provided with means (10) to retain the plurality of substantially identical bodies (3) within the chamber means (2).

12. A fluid flow sensing device (1) as claimed in any one of the preceding claims, **characterised in that** an average diameter of each said substantially identical body (3) is between one third and one twentieth of a diameter of the chamber means (2), said diameter being taken orthogonally to an axis of the chamber means (2) extending between its respective first and second opening means.

13. A fluid flow sensing device (1) as claimed in any one of the preceding claims, **characterised in that** the fluid flow sensing device (1) is so configured that said plurality of substantially identical bodies (3) may be removed and replaced with a second plurality of different substantially identical bodies (3) for example having a different diameter.

14. A fluid flow sensing device (1) as claimed in any one of the preceding claims, **characterised in that** the fluid comprises a gas and the fluid flow sensing device (1) comprises a gas flow sensing device (1).

15. Anaesthetic ventilator apparatus **characterised in that** it is provided with a fluid flow sensing device (1) as claimed in any one of the preceding claims.
